(19) Europäisches Patentamt
European Patent Office
Office européen des brevets

(11) Veröffentlichungsnummer: **0 363 449 B1**

(12) **EUROPÄISCHE PATENTSCHRIFT**

(45) Veröffentlichungstag der Patentschrift: **18.11.93**

(51) Int. Cl.5: **G01N 33/564**, G01N 33/531, C07K 3/28, C07K 15/00

(21) Anmeldenummer: **89902013.5**

(22) Anmeldetag: **15.02.89**

(86) Internationale Anmeldenummer: **PCT/CH89/00025**

(87) Internationale Veröffentlichungsnummer: **WO 89/07764 (24.08.89 89/20)**

(54) **VERFAHREN ZUR UNTERSUCHUNG VON ANTIKÖRPERN SPEZIFISCH FÜR RHEUMAKRANKHEITEN, INSBESONDERE CHRONISCHE POLYARTHRITIS SOWIE REAGENS HIERFÜR.**

(30) Priorität: **18.02.88 AT 389/88**

(43) Veröffentlichungstag der Anmeldung:
**18.04.90 Patentblatt 90/16**

(45) Bekanntmachung des Hinweises auf die Patenterteilung:
**18.11.93 Patentblatt 93/46**

(84) Benannte Vertragsstaaten:
**AT BE CH DE FR GB IT LI NL SE**

(56) Entgegenhaltungen:
**EP-A- 0 175 310**
**WO-A-60/4588**

**BIOLOGICAL ABSTRACTS/RRM, Band 35; W. HASSFELD et al., Nr. 53633&NUM;**

**JOURNAL OF BIOLOGICAL CHEMISTRY, Band 250, Nr. 9, 10 Mai 1964,The Americal Soc. of Biological Chemists (US); I. PET-TERSSON et al., Seiten 5907-5914&NUM;**

(73) Patentinhaber: **Smolen, Josef, Dr.**
**Cuviergasse 23**
**A-1130 Wien(AT)**

(72) Erfinder: **HASSFELD, Wolfgang**
**Joanellig. 5/2/20**
**A-1060 Wien(AT)**
Erfinder: **SMOLEN, Josef**
**Cuvierg. 23**
**A-1130 Wien(AT)**
Erfinder: **STEINER, Günter**
**Hafeng. 13/24**
**A-1030 Wien(AT)**

(74) Vertreter: **Müllner, Erwin, Dr. et al**
**Patentanwälte,**
**Dr. Erwin Müllner,**
**Dipl.-Ing. Werner Katschinka,**
**Dr. Martin Müllner,**
**Postfach 159,**
**Weihburggasse 9**
**A-1010 Wien (AT)**

CLIN. EXP. IMMUNOL., Band 86, 1991; Seiten
71-78&NUM;

**Beschreibung**

Die Erfindung betrifft ein Verfahren zur Untersuchung menschlicher oder tierischer Körperflüssigkeiten, wie eines Serums, auf das Vorhandensein von Antikörpern, spezifisch für Krankheiten des rheumatischen Formenkreises und entzündliche Erkrankungen, insbesondere für chroische Polyarthritis, wobei eine Probe der zu untersuchenden Körperflüssigkeit mit einem Reagens aus einem Zellkernextrakt in Kontakt gebracht wird.

Etwa die Hälfte der Bevölkerung leidet zu verschiedenen Zeiten des Lebens an rheumatischen Erkrankungen. Bei etwa 10% davon verlaufen sie entzündlich und meistens chronisch. Diese Krankheiten führen nicht nur zu Gelenksentzündungen, Schmerzen und Bewegungsbeeinträchtigung, sie können auch, wie z.B. im Falle der chronischen Polyarthritis (cP), eine Verkrüppelung bis hin zur Immobilisierung verursachen oder führen bei Beteiligung lebenswichtiger Organe sogar zu raschem Tod.

Da bei vielen entzündlichen Erkrankungen ähnliche klinische Symptome auftreten können, lässt sich allein durch die ärztliche Untersuchung, durch apparative Methoden, wie z.B. Röntgenuntersuchungen, und durch herkömmliche Laboruntersuchungen die Diagnose häufig nicht eindeutig stellen. Eine klarere Differenzierung dieser Krankheiten erlauben aber zusätzliche serologische Analysen.

Aus diesem Grund haben serologische Untersuchungen in der rheumatologischen Diagnostik breiteste Anwendung gefunden. Dabei werden Seren auf das Vorhandensein von Antikörpern untersucht, die der Körper im Fall dieser Erkrankungen gegen körpereigene Substanzen bildet. Diese Antikörper werden aus diesem Grund auch als "Autoantikörper" bezeichnet.

In der Diagnostik haben sich vor allem zwei Gruppen von Autoantikörpern als wichtig erwiesen: Rheumafaktoren (RF) und anti-nukleäre Antikörper (ANA). Rheumafaktoren sind Autoantikörper gegen körpereigene Immunglobuline, also anti-Immunglobulin-Antkörper, antinukleäre Antikörper sind Autoantikörper gegen Strukturen körpereigener Zellkerne.

Leider sind weder Rheumafaktoren noch antinukleäre Antikörper spezifisch für eine bestimmte Erkrankung. So treten Rheumafaktoren zwar bei 70 bis 80% aller cP-Patienten auf, doch kommen sie auch bei viralen, parasitären und chronisch-bakteriellen Erkrankungen vor, bei chronischen Leber- und Lungenerkrankungen, bei manchen krebsartigen Krankheiten sowie bei anderen entzündlichen rheumatischen Erkrankungen, wie z.B. dem SLE (ca. 30% der Patienten), der Sklerodermie (ca. 25%) und dem Sjögren Syndrom (bis zu 70%). Darüber hinaus treten sie mit zunehmendem Alter auch in bis zu 10% der gesunden Bevölkerung auf.

Dazu kommt noch, dass bei etwa 20 bis 30% der cP-Patienten Rheumfaktoren nicht nachgewiesen werden können. Diese Gruppe von seronegativen cP-Patienten muss daher klinisch nicht nur gegen jene rheumatischen Krankheiten differenziert werden, bei denen Rheumafaktoren gelegentlich vorkommen können, sondern auch gegen jene, die charakteristischerweise mit einem negativen Rheumafaktornachweis einhergehen, wie z.B. die Psoriasisarthritis, die reaktive Arthritis, der Morbus Reiter, sowie gelegentlich gegen degenerative Gelenkserkrankungen, die Arthrosen.

Auch antinukleäre Antikörper können bei allen rheumatischen Autoimmunerkrankungen einschliesslich der cP, aber auch bei Patienten mit nicht-rheumatischen Krankheiten und gelegentlich bei gesunden Probanden nachgewiesen werden.

Versucht man, antigene Strukturen, gegen die diese antinukleären Antikörper gerichtet sein können, näher zu charakterisieren, wird eine bessere Zuordnung zu bestimmten Krankheitsbildern möglich. Manche dieser antinukleären Antikörper-Subtypen gelten heute sogar als pathognomonisch.

Aus der EP-A-313 156 (ein Stand der Technik nach Art.54(3)) ist ein Antigen mit einem Molekulargewicht von 31.2kD, das in einem Verfahren zur Bestimmung von Autoimmun-Antikörpern eingesetzt wird, bekannt. Dieses Antigen ist als das Zellkernprotein $U_1$-snRNP-A identifiziert worden. Es reagiert spezifisch mit Seren von Patienten mit systemischem Lupus erythematosus (SLE) oder "Mixed Connective Tissue Disease" (MCTD) . Auch aus J Biol Chem 250 (1984) 5907-5914 ist bekannt, $U_1$-snRNP-Protein(Molekulargewicht 33kD) zur Bestimmung von Autoimmun-Antikörpern zu verwenden.

Ein Hilfsmittel zur Erkennung der cP war bisher ein Verfahren zum Nachweis von antinukleären Antikörpern gegen nukleäre Antigene aus Epstein-Barr-Virus (EBV) infizierten Zellen. Dieser antinukleäre Antikörper-Subtyp tritt gehäuft bei cP auf. Er ist aber leider auch in anderen Patientenpopulationen, z.B. Patienten mit floriden oder abgelaufenen EBV-Infektionen, nachweisbar und somit nicht spezifisch für cP.

Die vorliegende Erfindung bezweckt eine Verbesserung der Diagnosemöglichkeiten und stellt sich die Aufgabe, ein Verfahren zur Untersuchung menschlichen oder tierischen Serums auf das Vorhandensein von Antikörpern zu entwickeln, die für chronische Polyarthritis typisch sind.

Diese Aufgabe wird durch ein Verfahren der eingangs genannten Art erfindungsgemäß dadurch gelöst, daß das Reagens ein spezifisches Antigen bzw. eine spezifische Antigenfraktion mit einem Molekulargewicht von etwa 33kD enthält, das bzw. die mit in der Körperflüssigkeit von Patienten mit chronischer Polyarthritis (cP) enthaltenen Antikörpern, nicht aber mit in der Körperflüssigkeit von Patienten mit systemischem Lupus erythematosus (SLE) oder "Mixed Connective Tissue Disease" (MCTD) enthaltenen Antikörpern eine Bindungsreaktion ergibt bzw.ergeben.

Dieses Molekulargewicht ergibt sich bei Anwendung der SDS-Polyacrylamidgel-Elektrophorese unter denaturierenden und reduzierenden Bedingungen und RNAse-Behandlung des Extraktes und unter Verwendung von 14C-methylierten Proteinmolekulargewichtsstandards mit 14,3 bis 200 kD der Firma Amersham, Katalog Nr. CFA 626.

Im besonderen wurden spezifische Antigene, die zu Bindungsreaktionen mit cP-Antikörpern befähigt sind, mit einem Molekulargewicht von 20, 25, 31, 33 und 36 kD festgestellt, wobei das Antigen mit dem Molekulargewicht von 33 (RA33) das am häufigsten vorkommende Antigen ist.

Für dieses Antigen sind folgende weitere Merkmale chrakteristisch:
- gleiche Wanderungsgeschwindigkeit unter reduzierenden sowie unter nicht-reduzierenden Bedingungen;
- Stabilität im pH-Bereich von 1 bis 9 während 30-minütiger Inkubation;
- Beibehaltung der antigenen Eigenschaften während 30 Minuten bei 56°C und während 5 Minuten bei 96°C;
- Beibehaltung der antigenen Eigenschaften auch bei wiederholtem Auftauen von -70°C (mindestens dreimaliges Auftauen).

Die spezifisch gegen RA33 gerichteten antinukleären Antikörper werden nur in Seren von cP-Patienten gefunden, nicht bei Patienten mit anderen Autoimmunerkrankungen, wie aus der folgenden Tabelle 1 zu ersehen ist.

Tabelle 1

| Erkrankung | Anzahl der getesteten Seren | Nachweis von ANA gegen RA33 | Häufgikeit bekannter ANA*) |
|---|---|---|---|
| chronische Polyarthritis | 116 | 37 | - |
| gesunde Kontrolle | 40 | 0 | - |
| andere rheumatische Erkrankungen | 87 | 0 | - |
| davon: Psoriasisarthritis | 12 | 0 | - |
| M.Bechterew | 14 | 0 | - |
| Arthrosen | 12 | 0 | - |
| SLE | 30 | 0 | 22 Ro/SSA, 6 La/SSB, 6 Sm, 1 PCNA 1 snRNP, 2 rRNP |
| Sjögren Syndrom | 3 | 0 | 3 Ro/SSA, 3 La/SSB |
| Mixed Connective Tissue Desease (MCTD) | 5 | 0 | 5 sn RNP |
| Sklerodermie | 11 | 0 | 3 Scl 70 |

*) Die in der letzten Spalte der Tabelle 1 angeführten Abkürzungen sind aus der Literatur bekannte antinukleäre Antikörper.

Von 116 getesteten cP-Seren konnten in 37 Fällen (= 32%) Antikörper gegen RA33 mittels Immunoblot-Technik nachgewiesen werden. Wie ersichtlich, fiel der Test bei allen anderen Autoimmunerkrankungen negativ aus. Zusätzlich zu den angeführten Erkrankungen wurden noch Seren von Patienten mit Crest Syndrom, Polymyositis, M.Wegener, M.Whipple, chronischer EBV-Infektion untersucht. Auch dabei waren keine antinukleären Antikörper gegen RA33 nachweisbar.

RA33 ist nicht identisch mit anderen bekannten Auto-Antigenen, wie durch spezifische Referenzseren mittels verschiedener immunologischer Techniken, wie Immunoblot, ELISA und Ouchterlony Doppelimmundiffusion gezeigt werden konnte.

In Tabelle 2 sind jene Auto-Antigene aufgelistet, die auf mögliche Kreuzreaktivität zu RA33 untersucht wurden und deren Identität mit RA33 ausgeschlossen werden kann.

Tabelle 2

| Autoantigen | MG (kD) | Assoziation mit Erkrankung |
|---|---|---|
| Ro (SSA) | 57 | SLE, primärer Sjögren |
| La (SSB) | 50,43 | SLE, primärer Sjögren |
| Sm | 25,26 | SLE |
| snRNP (U1 RNP) | 68,34,32 | SLE, MCTD |
| Histone | 11-24,31 | SLE, drogeninduzierter LE |
| rRNP | 16/17,38 | SLE, drogeninduzierter LE |
| PCNA | 36 | SLE |
| SCL 70 | 70 | Sklerodermie |
| nukleäre Lamine | 60,70 | Sklerodermie |
| Jo-1 | 50 | Polymyositis |
| Centromere | 14,20,23,24 | Crest-syndrom |
| Laux | 83 | Sklerodermatomyositis |
| Ku | 70,80 | Sklerodermatomyositis |
| Rana | 80 | EBV-Infektionen, cP |

Das Reagens für die erfindungsgemäße Untersuchung kann hergestellt werden, indem
- Zellen menschlicher oder tierischer Herkunft homogenisiert werden, um die Zellkerne freizulegen,
- die abgetrennten Zellkerne mit einer hypotonischen Pufferlösung behandelt werden, um die Zellkerne zum Quellen zu bringen,
- anschliessend die Suspension mit einer hypertonischen Pufferlösung behandelt wird, um leicht lösliche Zellkerninhaltsstoffe in Lösung zu bringen,
- die Feststoffe durch Zentrifugieren abgetrennt werden und
- die überstehende Lösung als Reagens formuliert wird.

Bei einer vorteilhaften Variante dieses Verfahrens werden die abgetrennten Zellkerne gesammelt und in haltbare Form, insbesondere durch Tieffrieren, gebracht; die Extraktion wird erst nach Zwischenlagerung der Zellkernfraktion vorgenommen.

Die Formulierung als Reagens schliesst auch eine Fraktionierung ein, bei der die reaktiven Antigene z.B. durch chromatographische Trennverfahren isoliert und anschliessend in haltbare Form gebracht werden.

Die reaktiven Antigene können danach kovalent oder nicht kovalent an eine Matrix immobilisiert und mit Körperflüssigkeit in Kontakt gebracht werden. Die Antikörper können mittels nachfolgender Verfahren nachgewiesen werden.

1) ELISA:

Die Antigene werden non-kovalent an eine Kunststoffoberfläche, vorteilhafterweise an Mikrotiterplatten, gebunden. Dies geschieht durch mehrstündige Inkubation in Boratpuffer (pH 8,2 bei 4°C, 100 mmol/l Borsäure, 25 mmol/l Na-Tetraborat, 75 mmol/l NaCl).

Freie Bindungsstellen werden durch Inkubation mit Boratpuffer, der 3% fettarme Trockenmilch enthält, oder Boratpuffer, der 1% BSA enthält, abgesättigt. Nach mehreren Waschschritten erfolgt die Inkubation mit den im Boratpuffer verdünnten Seren (2 h, 37°C). Antikörper gegen RA33 werden mittels eines gegen den Fc-Teil von humanem IgG gerichteten Antikörpers, der mit alkalischer Phosphatase oder Peroxidase gekoppelt ist, nachgewiesen. Als Substrate für die Enzyme können p-Nitrophenylphosphat, o-Anisidin, o-Phenylendiamin oder ABTS dienen. Die Intensität der entstehenden Farbe ist direkt proportional der Bindung der Antikörper.

2) Radioimmunoassay (RIA):

In einem analogen Verfahren wird das Antigen an Polystyrol-Kugeln gebunden. Der Nachweis erfolgt ähnlich wie beim Western-Blot-Verfahren durch Inkubation mit einem gegen die Fc-Region des IgG gerichteten Kaninchen-Human-Antikörpers und anschliessende Inkubation mit 125J-markiertem Anti-Kaninchen-Antikörper Die Kugeln werden mehrmals gewaschen und die Menge an gebundener Radioaktivität bestimmt. Die gemessene Radioaktivität ist ein direktes Mass für das Vorhandensein der Antikörper.

3) Streifentest:

Die Antigene werden durch Auftropfen auf einen Träger (z.B. Nitrozellulose, Nylonmembran) immobilisiert und anschliessend mit Seren in Kontakt gebracht. Der Nachweis der Antikörper gegen RA33 erfolgt dann in gleicher Weise wie beim Western-Blot-Verfahren entweder radiochemisch oder Photometrisch in Analogie zu dem bei der ELISA-Technik beschriebenen Verfahren.

4) Agglutinationstest:

Bei diesem Verfahren werden die Antigene an Partikel, z.B. Latex, adsorbiert. Ein Tropfen des verdünnten Patientenserums wird auf einem Objektträger mit einem Tropfen der Latex-Suspension in Kontakt gebracht. Mit einem dünnen Stab wird gut gemischt und nach 2 bis 5 Minuten auf Agglutination geprüft und mit einem Kontrollserum verglichen. Durch entsprechende Verdünnung der positiven Seren kann der Antikörpertiter halbquantitativ erfasst werden.

In ähnlicher Weise können auch Erythrozyten mit Antigenen sensibilisiert und durch Antikörper zur Agglutination gebracht werden.

5) Doppelimmundiffusion (DID):

Bei dieser Methode wird die Präzipitation von Antigen-Antikörper-Komplexen in einem Trägermedium, vorzugsweise Agarose, nachgewiesen. Dazu werden in die Agarose Löcher gestanzt, wobei das Antigen in das mittlere Loch, die Seren in die radialen Löcher gefüllt werden. Eine Präzipitationslinie deutet auf das Vorhandensein von Antikörpern hin. Ihre Identität kann durch Vergleich mit monospezifischen Kontrollseren überprüft werden.

Eine besonders günstige Variante des erfindungsgemässen Verfahren zur Untersuchung der Seren besteht darin, dass
- die im Zellkernextrakt enthaltenen Inhaltsstoffe nach ihrer Wanderungsgeschwindigkeit in einem Trägermedium elektrophoretisch aufgetrennt werden, um neben verschiedenen aufgetrennten Fraktionen das in Anspruch 1 definierte spezifische Antigen bzw. die in Anspruch 1 definierte spezifische Antigenfraktion festzulegen,
- die elektrophoretisch aufgetrennten Fraktionen von dem Trägermedium auf eine Nitrocellulosemembran übertragen werden
- die Membran nach Absättigung freier Bindungsstellen mit der zu untersuchenden Körperflüssigkeit in Kontakt gebracht wird, worauf im Falle einer positiven Immunreaktion des Antigens bzw. der Antigenfraktion mit einem in der Körperflüssigkeit vorhandenen Antikörper die Bindungsreaktion mittels eines mit diesem Antikörper reagierenden markierten Reagens sichtbar gemacht wird.

Das erfindungsgemäss Verfahren zur Untersuchung auf antinukleäre Antikörper gegen RA33 ermöglicht weiters in Kombination mit dem Nachweis auf Rheumafaktoren zusätzliche für die Diagnose wertvolle Aussagen. So ist aus Tabelle 3 ersichtlich, dass bei mehr als der Hälfte jener Patienten mit cP, in deren Seren keine Rheumafaktoren nachgewiesen werden konnten, sehr wohl Antikörper gegen RA33 gefunden werden.

## Tabelle 3

Auftreten von Rheumafaktoren (RF) und Antikörpern gegen RA33 bei 50 Patienten mit chronischer Polyarthritis. Die Rheumafaktoren wurden mittels Waaler Rose- und Latex-Test bestimmt.

|  | $RF_{neg}$ | $RF_{pos}$ |
|---|---|---|
| RA33 pos | 8 | 12 |
| RA33 neg | 7 | 23 |

Antikörper gegen "RANA" (Rheumatoid Arthritis Nuclear Antigen), ein EBV-assoziiertes nukleäres Antigen mit einem Molekulargewicht von 80 kD, treten bei der cP übrigens mit ähnlicher Häufigkeit auf wie die Rheumafaktoren, aber auch bei einer Vielzahl anderer Erkrankungen sowie bei einem relativ hohen Prozentsatz Gesunder. Tabelle 4 zeigt, dass von 16 untersuchten Patienten mit cP annähernd 70% Antikörper gegen RANA und 50% Antikörper gegen RA33 aufwiesen. Bei zwei der RA33 positiven Patienten konnten keine Antikörper gegen RANA festgestellt werden.

Tabelle 4

| Auftreten von Antikörpern gegen RA33 und RANA (Rheumatoid Arthritis Nuclear Antigen), ein EBV assoziiertes Protein bei 16 Patienten mit chronischer Polyarthritis. | | |
|---|---|---|
| | $RA33_{pos}$ | $RA33_{neg}$ |
| RANA pos | 6 | 5 |
| RANA neg | 2 | 3 |

Eine Möglichkeit für die Herstellung der Zellkernextrakte wid im folgenden Beispiel beschrieben:

Als Zellen können z.B. Leukozyten oder Zellinien verwendet werden. Zellinien haben den Vorteil, dass sie eine weitgehend homogene Zellpopulation darstellen, müssen aber über einige Tage unter den für sie spezifischen Bedingungen kultiviert werden, bis sie zu einer Dichte von 1 bis 2 Millionen pro Milliliter gewachsen sind.

Die Zellen wurden in diesem Beispiel durch Zentrifugieren (15 Minuten bei 3000 g) geerntet, einmal in isotonischem Puffer (10 mmol/l Hepes, pH 7,9, 140 mmol/l KCl, 1,5 mmol/l $MgCl_2$, 0,5 mmol/l Dithiothreitol (DTT), 0,5 mmol/l Phenylmethylsulfonylfluorid (PMSF)) gewaschen und anschliessend im selben Puffer resuspendiert (12 ml Puffer für 10E9 Zellen). Die Zellen wurden in einem Glashomogenisator vorsichtig homogenisiert, bis die Zellkerne frei von Cytoplasma waren. Dies wurde durch Mikroskopie des Zellhomogenates überprüft. Im allgemeinen ist eine Homogenisierzeit von 30 Minuten ausreichend.

Ds Homogenisat wurde 3 Minuten bei 3000 g zentrifugiert, der Zellmembranen und Mikrosomen enthaltende cytoplasmatische Ueberstand entfernt und das hauptsächlich aus Zellkernen bestehende Zentrifugat noch einmal 30 Minuten bei 20.000 g zentrifugiert. Der Ueberstand wurde verworfen und die Kerne in hypotonischem Puffer (20 mmol/l Hepes, pH 7,9, 1,5 mmol/l $MgCl_2$, 0,4 mmol/l Aethylendiamintetraessigsäure (EDTA), 0,5 mmol/l DTT, 25% v/v Glycerin) suspendiert (1 ml Puffer für 10E9 Kerne). Anschliessend wurden sie 30 Minuten auf Eis unter leichtem Rühren inkubiert. Dann wurde dasselbe Volumen an hypertonischem Puffer (20 mmol/l Hepes, pH 7,9, 3 mmol/l $MgCl_2$, 0,4 mmol/l EDTA, 0,5 mmol/l DTT, 880 mmol/l $NH_4Cl$, 25% v/v Glycerin) zugesetzt, weitere 45 Minuten auf Eis unter Rühren inkubiert und danach 20 Minuten bei 50.000 g zentrifugiert. Der Ueberstand stellt den löslichen Kernextrakt dar und wurde nach Dialyse gegen Dialysepuffer (20 mmol/l Hepes, pH 7,9, 100 mmol/l KCl, 0,4 mmol/l EDTA, 0,5 mmol/l DTT, 1 mmol/l PMSF, 20% v/v Glycerin) in Aliquoten bei -70°C gelagert und in weiterer Folge zum Nachweis von Autoantikörpern eingesetzt.

Nach einer alternativen Ausführungsform können die Kerne auch nach Resuspension in hypotonischem Puffer bei -20°C eingefroren werden. Nach gewünschter Lagerungszeit werden die Zellkerne in der angegebenen Weise mit hypertonischem Puffer extrahiert. Durch dieses modifizierte Verfahren werden zusätzliche Antigene aus den Zellkernen extrahiert.

Die Antigenität der Komponenten des Kernextraktes kann wie in diesem Beispiel - mittels der Immunblot-Technik (Western-Blot) nachgewiesen werden. Dazu wurde der Extrakt mit isotonischem Puffer auf eine Proteinkonzentration von etwa 10 mg/ml verdünnt, mit vierfach-Probenpuffer (einfach-Probenpuffer: 125 mmol/l Tris-HCl, pH 8,3, 2,3% Natriumdodecylsulfat (SDS), 5% Mercaptoäthanol, 10% Glycerin) versetzt und der Elektrophorese unterzogen. Zu diesem Zwecke wurde ein 12%iges SDS-Polyacrylamidgel, pH 8,3, mit einem 5%igen Stackinggel, pH 6,8, und ein Elektrophoresepuffer (25 mmol/l Tris, 192 mmol/l Glycin, 0,1% SDS, pH 8,3) verwendet.

Die Proteine wurden 14 Stunden bei einer Stromstärke von 20 mA unter Wasserkühlung nach ihren relativen molaren Massen (MG) aufgetrennt und anschliessend auf eine Nitrozellulosemembran (NZ) transferiert (geblottet). Der Transfer wurde in einer Blotting-Kammer 150 Minuten bei einer konstanten Stromstärke von 250 mA im elektrischen Feld des Transferpuffers (25 mmol/l Tris, 192 mmol/l Glycin, 20% v/v Methanol, pH 8,3) durchgeführt.

Im Anschluss daran wurde die Nitrozellulose in Streifen geschnitten und ihre unspezifischen Bindungsstellen mit Borat-Puffer (100 mmol/l Borsäure, 25 mmol/l Na-Tetraborat, 75 mmol/l NaCl, pH 8,2), der 3% fettarme Trockenmilch enthält (BM-Puffer), 30 Minuten bei Raumtemperatur abgesättigt. Danach wurde die NZ mit den zu untersuchenden Humanseren 90 Minuten bei Raumtemperatur unter leichtem Schwenken inkubiert, wozu die Seren 1:10 mit BM-Puffer verdünnt wurden. Nicht an Antigene gebundene Serumantikörper wurden durch dreimaliges Waschen mit Boratpuffer entfernt.

Die NZ wurde dann mit einem gegen die Fc-Region des IgG gerichteten Kaninchen-anti-Human-Antikörper (1:500 mit BM-Puffer verdünnt) eine Stunde bei Raumtemperatur unter leichtem Schwenken inkubiert. Nach einem Waschschritt mit Boratpuffer erfolgte die Inkubation der NZ mit einem (125J)-markierten Esel-anti-Kaninchen-Ig Antikörper (spezifische Aktivität 185 bis 740 kBq/$\mu$g, Verdünnung 1:500 mit BM-Puffer), um die Bindungsstellen der humanen Auto-Antikörper an die NZ-Streifen mittels Autoradiographie sichtbar machen zu können. Inkubiert wurde eine Stunde bei Raumtemperatur unter leichtem Schwenken.

Nach dreimaligem Waschen mit Boratpuffer wurden die NZ-Streifen getrocknet, auf Karton geklebt und in einer Röntgenkassette an einem Autoradiographiefilm exponiert. Nach der Entwicklung wiesen die geschwärzten Stellen (Banden) auf die Bindungsstellen des radioaktiven Antikörpers hin und somit indirekt auf das Vorhandensein von gegen Antigene des Zellkernextraktes gerichtete Antikörper. Zur Feststellung des Molekuklargewichtes dienten (14C)-markierte Proteine mit definiertem Molekulargewicht (MG-Marker), die gemeinsam mit dem Extrakt auf NZ geblottet wurden.

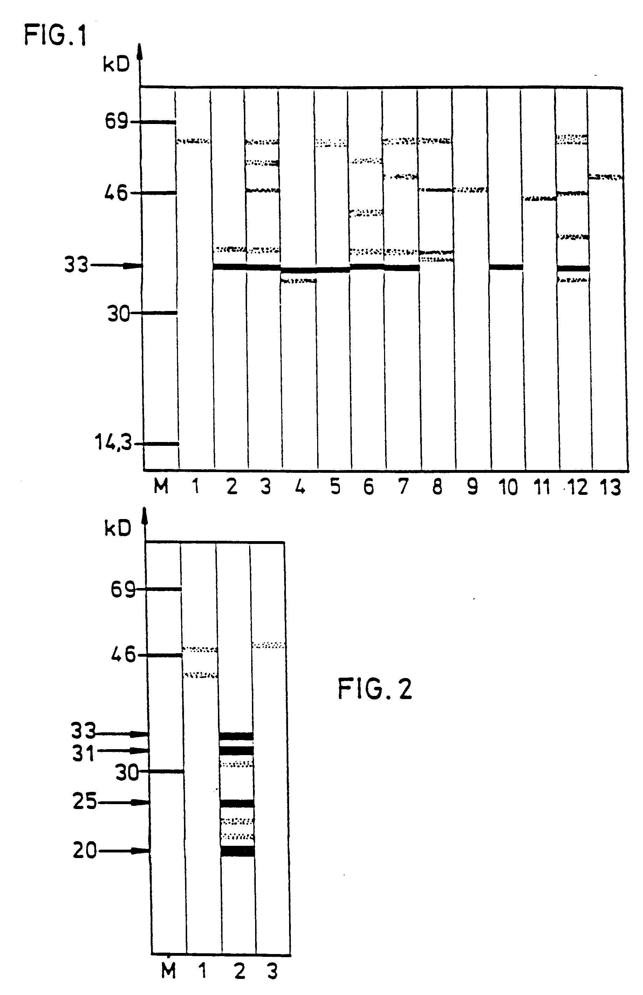

Das Ergebnis ist im beiliegenden Autoradiogramm (Fig. 1) dargestellt. Es zeigt die autoradiographischen Banden von 13 Proben, entsprechend den Spuren 1 bis 13 und der Spur des Molekulargewicht-Markers (M). Die Spur 1 wurde mit menschlichem Normalserum als Blindprobe erhalten. Die Spuren 2 bis 7, 10 und 12 stammen von cP-Seren, die Antikörper gegen RA33 enthalen. Die geschwärzten Stellen (Banden) sind bei etwa 33 kD zu finden. Die Spuren 8, 9, 11 und 13 wurden von cP-Seren erhalten, in denen kein Antikörper gegen RA33 vorhanden war.

Gemäss der früher angeführten abgeänderten Ausführungsform wurde die Zellkernfraktion vor der Extraktion des antigenen Materials tiefgefroren. Dabei zeigte sich, dass im Autoradiogramm neben der Bande bei 33 kD noch weitere Banden bei 20, 25, 31 und 36 kD sichtbar wurden. Das geht aus Fig. 2 hervor.

Mit M ist in Fig. 2 die Spur des Molekulargewicht-Markers bezeichnet. Die Spur 1 wurde mit menschlichem Normalserum als Blindprobe erhalten, die Spur 2 stammt von einem cP-Serum, in dem zusätzlich zu Antikörpern gegen RA33 noch solche gegen Antigene mit 20, 25, 31 und 36 kD enthalten sind, und die Spur 3 bezieht sich auf ein cP-Serum, in dem keine Antikörper gegen Antigene im Bereich von 15 bis 40 kD nachgewiesen werden konnten.

Die zusätzlichen Banden erscheinen übrigens immer gemeinsam mit jener, die dem Antigen RA33 zuzuordnen ist.

## Nachweis von Anti-RA33-Immunglobulinsubklassen

Mit Hilfe des Immunoblot-Verfahrens können prinzipiell auch Immunglobulinsubklassen durch Wahl geeigneter Zweitreagenzien nachgewiesen werden, und dies wurde für Anti-RA33 durchgeführt. Zu diesem Zweck wurde ein bis hin zur Inkubation mit den Patientseren identes Immunoblot-Protokoll durchgeführt; danach wurde eine Inkubation der Nitrouellulosestreifen mit gegen IgG-Fc-Region gerichtetem Kaninchen-Antikörper (affinitätschromatographisch gereinigt, Jackson Research Laboratories, Cochranville/USA) bzw. ein gegen die IgM-U-Kette gerichtetem, affinitätschromatographisch gereinigtem Kaninchen-Antikörper (Jackson). Nach anschliessendem Waschen wurden die Nitrozellulosestreifen 40 Minuten bei Raumtemperatur mit einem gegen Kaninchen Immunglobulin gerichteten, 125-J-markierten Antikörper (Donkey-anti-Rabbit-Ig affinitätschromatographisch gereinigt) inkubiert. Die restlichen Schritte waren identisch mit dem auf Seite 14 angeführten Protokoll. Die Ergebnisse dieser Untersuchungsserie erbrachten, dass Anti-RA33 sowohl der Klasse IgG als auch der Klasse IgM angehören kann.

## Nachweis von Anti-RA33 in Synovialflüssigkeiten (Gelenkflüssigkeiten):

In weiteren Untersuchungsschritten wurde die Frage aufgeworfen, ob Anti-RA33 nur im Serum von Patienten mit chronischer Polyarthritis oder auch in Synovialflüssigkeiten auftreten könne; zu diesem Zweck wurde ein Immunoblot-Protokoll entsprechend den obigen Ausführungen durchgeführt, wobei allerdings statt Sera der Patienten Synovialflüssigkeiten eingesetzt wurden. Von den 6 untersuchten Synovialflüssigkeiten konnten bei 3 Anti-RA33-Antikörper gefunden werden.

**Patentansprüche**

1. Verfahren zur Untersuchung menschlicher oder tierischer Körperflüssigkeiten, wie eines Serums, auf das Vorhandensein von Antikörpern, spezifisch für Krankheiten des rheumatischen Formenkreises und entzündliche Erkrankungen, insbesondere für chronische Polyarthritis, wobei eine Probe der zu untersuchenden Körperflüssigkeit mit einem Reagens aus einem Zellkernextrakt in Kontakt gebracht wird, dadurch gekennzeichnet, daß das Reagens ein spezifisches Antigen bzw. eine Spezifische Antigenfraktion mit einem Molekulargewicht von etwa 33 kD enthält, das bzw. die mit in der Körperflüssigkeit von Patienten mit chronischer Polyarthritis (cP)enthaltenen Antikörpern, nicht aber mit in der Körperflüssigkeit von Patienten mit systemischem Lupus erythematosus (SLE) oder "Mixed Connective Tissue Disease" (MCTD) enthaltenen Antikörpern eine Bindungsreaktion ergibt bzw. ergeben.

2. Verfahren nach Anspruch 1, dadurch gekennzeichnet, daß
   - die im Zellkernextrakt enthaltenen Inhaltsstoffe nach ihrer Wanderungsgeschwindigkeit in einem Trägermedium elektrophoretisch aufgetrennt werden, um neben verschiedenen aufgetrennten Fraktionen das in Anspruch 1 definierte spezifische Antigen bzw. die in Anspruch 1 definierte spezifische Antigenfraktion festzulegen,
   - die elektrophoretisch aufgetrennten Fraktionen von dem Trägermedium auf eine Nitrocellulosemembran übertragen werden
   - die Membran nach Absättigung freier Bindungsstellen mit der zu untersuchenden Körperflüssigkeit in Kontakt gebracht wird, Worauf im Falle einer positiven Immunreaktion des Antigens bzw. der Antigenfraktion mit einem in der Körperflüssigkeit vorhandenen Antikörper die Bindungsreaktion mittels eines mit diesem Antikörper reagierenden markierten Reagens sichtbar gemacht wird.

**Claims**

1. Method of testing human or animal body fluids, such as a serum, for the presence of antibodies specific for illnesses of the rheumatic form and inflammatory diseases, particularly for chronic polyarthritis, in which a specimen of the body fluid to be tested is placed in contact with a reagent from a cell-nucleus extract, characterised in that the reagent contains a specific antigen or a specific antigen fraction with a molecular weight of about 33kD, which produces a bonding reaction with antibodies contained in the body fluid of patients with chronic polyarthritis (CP), but not with antibodies contained in the body fluid of patients with systematic lupus erythematosus (SLE) or "Mixed Connective Tissue Disease" (MCTD).

2. Method according to claim 1, characterised in that
   - the components contained in the cell-nucleus extract are electrophoretically separated according to their migration rate in a carrier medium, in order to fix the specific antigen defined in claim 1 or the specific antigen fraction defined in claim 1 against different separated fractions,
   - the electrophoretically separated fractions are transferred from the carrier medium to a nitrocellulose membrane
   - the membrane, after saturation of free bonds, is placed in contact with the body fluid to be tested, whereupon in the case of a positive immunoreaction of the antigen or the antigen fraction with an antibody present in the body fluid the bonding reaction is made visible by means of a tagged reagent reacting with this antibody.

**Revendications**

1. Procédé pour l'examen de liquide humoral d'origine humaine ou animale, tel que sérum, relativement à la présence d'anticorps spécifiques des maladies à symptômatologie rhumatismale et des maladies de type inflammatoire, en particulier de la polyarthrite chronique, dans lequel un prélèvement de l'humeur à analyser est mis en contact avec un réactif obtenu à partir d'un extrait de noyau cellulaire, caractérisé en ce que le réactif contient un antigène spécifique, ou une fraction d'antigènes spécifiques dont le poids moléculaire est d'environ 33 kD, cet antigène ou fraction d'antigènes donnant lieu à une réaction de fixation avec les anticorps contenus dans les liquides humoraux de patients atteints de polyarthrite chronique (cP) mais ne donnant par contre aucunement lieu à réaction avec les anticorps contenus dans les humeurs des patients atteints de Lupus erythémateux systémique (SLE) ou de la connectivité mixte "Mixed Connective Tissue Disease" (MCTD).

**2.** Procédé selon la revendication 1, caractérisé en ce que:

- les composés contenus dans l'extrait de noyau cellulaire sont séparés dans un support par électrophorèse en fonction de leur vitesse de migration, dans le but de déterminer l'antigène spécifique ou la fraction spécifique d'antigènes tel que défini dans la revendication 1;
- les fractions séparées par électrophorèse sont transférées du support sur une membrane de nitrocellulose;
- la membrane après saturation des sites de fixation libres est mise au contact du liquide humoral à étudier, de sorte que dans le cas où une réaction immunitaire positive de l'antigène ou de la fraction d'antigènes avec un anticorps présent dans le liquide humoral est positive, la réaction de fixation soit rendue apparente du fait qu'un réactif marqué réagit avec cet anticorps.

FIG.1

FIG.2